# EUROPEAN PATENT SPECIFICATION

(11) **EP 4 282 335 B1**
(45) Date of publication and mention of the grant of the patent: **03.09.2025**
(21) Application number: 22175192.8
(22) Date of filing: 24.05.2022
(51) Int. Cl.: A61B 6/00, G06T 3/00

(54) **COMPUTERIZED RADIOGRAPHIC IMAGING**
RECHNERGESTÜTZTE RADIOGRAFISCHE ABBILDUNG
IMAGERIE RADIOGRAPHIQUE INFORMATISÉE

(43) Date of publication of application: 29.11.2023
(73) Proprietor: Sirona Dental Systems GmbH, 64625 Bensheim (DE); DENTSPLY SIRONA Inc., York, PA 17401 (US)
(72) Inventor: Maur, Susanne, 64625 Bensheim (DE); Braun, Tim, 64625 Bensheim (DE)
(74) Representative: Aldridge, Henry Alexander

(56) References cited:
- WO-A2-2022/072181

## Description

### TECHNICAL FIELD

The present teachings relate generally to computer-implemented methods and products for medical radiographic imaging, more particularly to dental radiography, especially X-ray based imaging.

### BACKGROUND

Radiological or radiographical medical imaging is used for viewing internal form of a patient's anatomy. Particularly in the domain of dental health, dental X-rays and computed tomography ("CT") scans of oral anatomies are commonly taken. Most such scans nowadays are digital in nature. Scan output obtained from an X-ray or CT system can be used for a variety of purposes, e.g., for supporting diagnosis or treatment. It is also known to generate a three-dimensional digital model or impression of a patient's anatomy using radiological scan data. Such a model can allow viewing the anatomy of the patient in a more intuitive form.

Scan output in modern radiographic systems is digital in nature which allows digital processing of the scan data. Data-driven techniques can also be applied to the scan output, however, obtaining sufficient, good quality and diverse training data for training data-driven logic can be a challenge.

The applicant has realized that there is a need for improved methods and systems which can allow better leveraging of data-driven techniques on radiological data.

Reference is made to WO2022072181A2 This document shows a computer-implemented method which includes providing three-dimensional data and volume annotation data with a transformation into two-dimensional data.

### SUMMARY

At least some of the limitations associated with the foregoing can be overcome by the subject matter of the accompanying independent claims. At least some of the additional advantageous alternatives will be outlined in the dependent claims.

Especially for supervised training, validation, or evaluation of an artificial intelligence ("AI") based system which identifies relevant aspects, such as outlines of anatomical features in medical images, a large amount of data may be required, for example, in the form of data pairs. A data pair comprises a radiological image, e.g., an X-ray image; and annotation data which comprises associated annotations. Each annotation usually describes a desired output result (such as the marked tooth outlines). The creation of such large amount of data or data pairs can be time-consuming and expensive. Annotation is usually performed by manual annotators.

By inventive effort the applicant has realized that existing radiological volumes which have been generated based on radiological scans of different patients can be leveraged for providing training data for data-driven techniques. Pursuant to the present teachings, a radiological volume, in a suitable form such as digital volume tomography ("DVT") volume, computed tomography ("CT") volume or cone beam computed tomography ("CBCT") volume, may be transformed to images of a predetermined modality, such as DVT image, panoramic image, intraoral image, cephalometric image, bitewing image or temporomandibular joint ("TMJ") image. Moreover, annotation data from the radiological volume are automatically processed and provided as appropriate for the images of the predetermined modality. These data can be used for configuring or improving data-driven logics.

There can be several advantages with doing so, for example, preexisting data (e.g., radiological volumes and their annotations) can be leveraged for the purpose of providing the training data or data pairs, rather than collecting new data, especially for training purposes. The training data generated pursuant to the present teachings can be tailor made to include various variations which may also include non-ideal behavior such as artifacts or distortion that sometimes manifests in certain radiological exposures. This can improve diversity and quality of the training data, moreover a data-driven logic trained using the proposed training data can also be made more robust by being able to differentiate between occurrence of said non-ideal behavior from a real anatomical situation which may exist in a patient being diagnosed. Moreover, certain data such as artefact ridden images can be technically very difficult to annotate. It may entail a substantial manual effort to annotate such data. Thus, quality and/or reliability of the data-driven logics trained and/or validated with the proposed training data can also be improved.
More specifically, when viewed from a first perspective, there can be provided a computer-implemented method for improving a medical radiological process, which method comprises:
- providing a three-dimensional radiological volume;
- providing volume annotation data; wherein the volume annotation data comprise a plurality of annotations, each annotation being indicative of an anatomical feature in the radiological volume,
- transforming at least a part of the radiological volume to a radiological projection; wherein the radiological projection is a two-dimensional or planar representation of a part of the radiological volume,
- automatically providing, using the volume annotation data, projection annotation data for the radiological projection; wherein the projection annotation data comprise at least some of the annotations present in the volume annotation data.

Thus, one or more correspondences are established between the anatomical features in the radiological volume and their related features present in the radiological projection, thereby resulting in the radiological projection being automatically annotated with the projection annotation data. This radiological projection, and preferably a plurality of radiological projections, along with their associated projection annotation data can be provided as a dataset which may be used for configuring a data-driven logic, which can include, training and/or validating or any sort of improvement of the data-driven logic. In many cases, the volume annotation data may already be existing for the radiological volume. These data can thus be efficiently utilized to automatically provide the projection annotation data for the radiological projection. This also prevents a need to annotate the radiological projection individually. The advantage can be more appreciated in cases where different radiological projections are generated from different perspectives of the radiological volume. Each of these radiological projections can thus have proper annotations dependent upon the anatomical features which are present when observed from said perspective of the respective radiological projection. The applicant has found the teachings especially suitable for the radiological volumes provided via radiographic imaging especially in the dental domain, more particularly with X-ray based imaging, however, the teachings can be applicable for volume models provided via other radiological principles as well. In the rest of the disclosure, without affecting the scope or generality of the present teachings, examples will be discussed with reference to oral anatomy.

It shall be appreciated that each of the annotations automatically provided in the projection annotation data is indicative of those one or more of the anatomical features which are also present in the radiological projection.

"Radiological volume" or "volume data" refers to a dataset or volume model representing an anatomy of a patient. More specifically, the radiological volume relates to craniofacial anatomy or oral anatomy of a patient. As non-limiting examples, the radiological volume may be in the form of computed tomography ("CT") volume, cone beam computed tomography ("CBCT"), digital volume tomography ("DVT") volume, or their likes. As a further non-limiting example, the radiological volume may be in the form of a CBCT volume of a patient's oral anatomy. As a further non-limiting example, the radiological volume describes properties of X-ray absorption and/or density of anatomical structures of the patient's craniofacial anatomy.

The radiological volume is thus a three-dimensional ("3D") digital record or impression of a plurality of anatomical features of the patient.

In some cases, the radiological volume may also comprise, or it may be linked to data from other modalities, such as intraoral scan, panoramic scan ("PAN"), cephalometric scan ("CEPH"), etc. Furthermore, in some cases, the radiological volume may even be annotated based on data from other modalities. Thus, in some cases, the volume annotation data may include data from other modalities.

"Volume annotation data" refer to data or one or more datasets which comprise a plurality of annotations for the respective anatomical features which are recorded in the radiological volume. Thus, each of the annotations is indicative of their respective anatomical feature in the radiological volume. As a few non-limiting examples, one or more of the annotations may be a specific tooth (e.g., recognized via a number, name or location), and/or one or more of the annotations may be a specific pathological condition (e.g., a fracture, cavity, missing tooth, caries, inflammation or their likes) and/or one or more of the annotations may be a specific nerve channel and/or one or more of the annotations may be a specific artificial object (e.g., a dental bridge, implant, crown, or their likes). The annotations for example may be in the form of a point location and/or in the form of an outline which is provided around a specific anatomical feature.

As non-limiting examples, the volume annotation data may be in the form of, or it may comprise, a voxel segmentation mask. Thus, the volume annotation data may comprise one or more voxel segmentation masks and/or one or more outlines and/or one or more point landmarks, and/or the projection annotation data comprise one or more pixel segmentation masks and/or one or more outlines and/or one or more point landmarks.

The volume annotation data may be a part of the radiological volume and/or it may be provided as separate one or more datasets, e.g., by linking to the radiological volume. Accordingly, the volume annotation data may or may not be stored together with the radiological volume. For example, the volume annotation data may be stored at least partially at another memory storage or location as compared to where the radiological volume is stored.

"Projection annotation data" refer to one or more datasets comprising at least some of the annotations present in the volume annotation data. More specifically the projection annotation data are indicative of those one or more of the anatomical features which are present in the radiological projection. In some cases, the projection annotation data may also comprise one or more additional annotations resulting from introducing one or more non-idealities. It shall be appreciated that any projection annotation data of any specific radiological projection from the plurality of radiological projections may or may not comprise an annotation. This is dependent on the kind of radiologic projection as it will be discussed later. Thus, it is possible that in some cases all radiological projections have annotations, while in other cases some, but not all, of the radiological projections have annotations in their respective projection annotation data. This will depend upon whether in a given radiological projection an anatomical feature and/or artifact which is annotated is present or not. If an anatomical feature annotated in the radiological volume is also present in a given radiological projection, the projection annotation data for that radiological projection will also include annotation for said anatomical feature. Similarly, if a radiological projection includes an artifact which has an annotation, the projection annotation data of the radiological projection will also include the annotation for the artifact. So, if an artifact is already present in the radiological volume and an annotation exists for the preexisting artifact, the preexisting annotation may also be included in the projection annotation data of the radiological projection. Alternatively, or in addition, the preexisting annotation may not exist, in which case the projection annotation data may be updated with additional annotations comprising information of the preexisting artifacts. In the latter case, sometimes the preexisting artifact may not be visible in the radiological volume, however it may manifest itself in the simulated radiological image as a result of the transformation process. In this case, the image annotation data may be updated with additional annotations comprising information of the preexisting artifacts.
According to an aspect, the method also comprises:
- reconstructing, from a plurality of radiological projections, one or more simulated radiological images;
- generating, using the projection annotation data, image annotation data for the simulated radiological image; wherein the image annotation data of at least one of the simulated radiological images comprise at least some of the annotations present in the volume annotation data.

Detection of artefacts or a successful detection of anatomical features despite artefacts being present in images can be very advantageous. In some types of images, artefacts such as motion and metal artefacts may only become visible after reconstruction. A dental panoramic image or orthopantomogram ("OPG") or PAN is such an example. The present teachings can thus provide a significant advantage of enabling artifact detection and/or a more robust detection of anatomical features in artefact ridden images. For example, a dataset obtained using the present teachings can result in such important improvements in the data-driven logics which have been processed or configured using the dataset.

"Reconstruction", in the context of reconstructing the simulated radiological image, refers to a process of building the simulated radiological image from the plurality of radiological projections. The process may be understood equivalent to a reconstruction process used to build images, e.g., OPGs or PANs, by combining projectional images acquired via an X-ray scan. Dependent upon the modality of the simulated radiological image, the reconstruction process may be panoramic image reconstruction, DVT reconstruction, CEPH reconstruction, bitewing image reconstruction, or any suitable image modality which is derivable from the radiological projections.

"Anatomical feature" refers to information related to a specific anatomy of the patient, e.g., craniofacial anatomy of the patient. For example, an anatomical feature may be information related to a specific tooth or a group of teeth. Thus, anatomical features may even refer to information related to any one or more intraoral structures such as, dentition, gingiva, nerve channel, extraction site, jawbone, and condyle. Alternatively, or in addition, anatomical features may be one or more artificial structures such as one or more fixation devices, e.g., bite-block, one or more dental replacements, e.g., dental crown, braces, veneer, bridge. Alternatively, or in addition, an anatomical feature may in some cases even be a scan body (or scanbody) or other natural and/or artificial structure attached to the jaw of the patient. Alternatively, or additionally, the anatomical feature may even be information related to a pathology or condition. As non-limiting examples, pathology or condition may be any one or more of: fracture of tooth or bone, caries, radiolucency, impaction, or any other characterizable state of the oral anatomy or any part thereof.

"Radiological projection" in the present context refers to a 2D image which is obtained from the radiological volume via the transformation operation as herein disclosed. As non-limiting examples, the radiological projections are virtual X-ray projections of the radiological volume. Those skilled in the art shall appreciate that certain imaging systems such as CBCT systems acquire a series of projectional images which are algorithmically combined to build a final image. As a non-limiting example, a typical X-ray system used for panoramic dental imaging includes a rotatable arm suspended at one end of a support column and an X-ray generator and an X-ray detector oppositely fixed to respective ends of the rotatable arm (C-arm). With a patient located between the generator and the detector during a scan, a plurality of X-ray exposures is performed with rotation of the arm such that the movements of the rotatable arm and thus also movements of the X-ray generator and the X-ray detector are synchronized in such a way that an image of an area of desired shape, for example the patient's dental arch, is captured via signal from the detector. The panoramic image is usually reconstructed from the plurality of exposures or projectional images. With reference to this example, the transformation operation would be equivalent to a simulated X-ray exposure.

Hence, it shall be appreciated that pursuant to the present teachings, the radiological volume is transformed to the plurality of radiological projections, which may be understood as using the radiological volume as a digital 3D object to computationally extract similar projectional images as a certain real-life radiological system would do from an equivalent real-life 3D object. The transformation may, for example, be performed via a computer simulated physical scan of the radiological volume. Hence, the plurality of radiological projections may be provided via an output of the simulated physical scan. The simulated physical scan may emulate mechanical, electronical and/or physical properties of a real-life radiological system, such as exposure time, arm radius, scan path, X-ray spectrum, properties of the X-ray detector, collimation, scan protocol, and so forth, but it may be simplified compared to a physically correct scan simulation. The transformation may involve simulating radiological behavior (e.g., X-rays) for the radiological volume with a material composition, or at least absorption properties associated with the anatomy or respective anatomical features.

Those skilled in the art shall appreciate that in the present context, a radiological projection is computationally derived from the radiological volume, rather than being captured via a radiological scanning system. Hence, the radiological projection is an image provided via an emulation logic which emulates a radiological scanning system. The emulation logic thus computes each radiological projection by considering the radiological volume as a 3D object scanned via the emulated radiological scanning system.

According to an aspect, in transforming the radiological volume to the plurality of radiological projections, one or more non-idealities may be introduced, even deliberately, e.g., via one or more non-ideal parameters or behavior models or a modification of the volume data by adding or removing or modifying anatomical features. As non-limiting examples, the non-idealities may be any one of: metal artifact, interfering structures, motion induced artifact, miscalibration induced artefact, malfunction induced artefact, distortion, noise, x-ray scatter, beam-hardening artefact, sensor artefact, or their likes. For example, during the transformation, metal object such as implants may be added at a certain location of the radiological volume. Additionally, or alternatively, material properties or size of a particular object may be manipulated, for example, a tooth crown may be computationally changed to a material with different density. Any similar or dissimilar non-idealities may thus be introduced to create radiological projections where the computed visual impact of these non-idealities mimics the impact one would encounter in an identical real scan situation. Thus, the non-ideality causes one or more simulated artifacts in at least some of the radiological projections or the simulated radiological image which is reconstructed using the radiological projections. Certain artifacts, like motion or metal artifacts, typically are more prominent in the reconstructed image as compared to the projection. The projection annotation data may thus be updated by including one or more additional annotations which provide information on the non-ideality which created the effect in the respective radiological projection. This can have the advantage that a data-driven logic processed using the data including such annotations can more reliably distinguish non-ideal features in images from anatomically relevant features.

The radiological projection is in general an image or planar representation of a part of the radiological volume. The radiological projection may be computationally captured from any suitable angle or projection of the radiological volume with considerations such as positioning a simulated X-ray source and simulated X-ray detector.

As a few non-limiting examples, the radiological projection may be an intraoral X-ray image, a single X-ray radiological projection, a one-shot CEPH image, or their likes which do not require a subsequent reconstruction, e.g., for human interpretation. In such cases, e.g., for intra-oral imaging the simulated detector is located inside the oral cavity of the volume, for example at or around the middle of the radiological volume.

Thus, in some cases, the radiological projection may be a narrow projection or a projection limited in its field of view or a projection limited in its dose such that a reconstruction is required using a plurality of such radiological projections to be able to view at least one anatomical feature, while in other cases the radiological projection may be a larger projection depicting at least one anatomical feature in a more complete manner. For example, typically, a panoramic image is reconstructed from narrow projections with low dose. Thus, features or even any artifacts may only become visible after the reconstruction process.

"Simulated radiological image" refers to any suitable image modality which is generated from reconstruction from a plurality of radiological projections obtained from the radiological volume. As a few non-limiting examples, the simulated radiological image may be a DVT image, a panoramic image, a CEPH image, or a bitewing image. In the present context, a simulated radiological image is computationally derived from the radiological volume, rather than being reconstructed from real-life projectional images captured via a radiological scanning system.

"Image annotation data" refer to one or more datasets derived from the projection annotation data related to the radiological projections which are used in reconstruction of the simulated radiological image. The image annotation data comprise a plurality of annotations for the respective anatomical features which are contained in the simulated radiological image. Thus, each of the annotations is indicative of their respective anatomical feature in the simulated radiological image. In some cases, the image annotation data may also comprise one or more additional annotations resulting from introducing one or more non-idealities during the projection process and/or the reconstruction process. As non-limiting examples, additional annotations may be any one or more of motion artefacts or metal artefacts or other image artefacts.

As it was discussed, the transformation in either case may be done via a simulated physical scan. Thus, the transformation of the radiological volume may involve:
- performing a simulated physical scan of the three-dimensional radiological volume;
- generating, from the simulated scan, the radiological projection or the plurality of radiological projections and/or simulated radiological images.

According to an aspect, the radiological volume is in the form of a DVT volume and at least one of the simulated radiological images is in the form of a DVT image and/or an orthopantomographic ("OPG") image, preferably a dental OPG image.

In some cases, one or more data processing operations may be performed prior to providing any of the images. Thus, the method may also comprise:
- performing a data processing operation on at least some of the radiological projections and/or the simulated radiological image; wherein, the data processing operation comprises any one or more of: contrast enhancement, providing artifact data, averaging, and filtering.

As it was also discussed previously, transforming the radiological volume to a plurality of radiological projections may further comprise including one or more non-idealities, wherein the non-ideality causes one or more simulated artifacts in at least some of the radiological projections and/or the simulated radiological image.

When viewed from another perspective, there can also be provided a dataset, or a non-transitory computer-readable storage medium storing the dataset, comprising at least one simulated radiological image and/or at least one radiological projection; and their corresponding image annotation data and/or projection annotation data; as generated in any of the herein discussed methods. For example, there can be provided a dataset, or a non-transitory computer-readable storage medium storing the dataset, comprising at least one simulated radiological image; and data associating each of the simulated radiological image to its corresponding image annotation data; said each of the simulated radiological images and their corresponding image annotation data being as generated in any of the methods herein disclosed.

As it was discussed, the datasets comprising the data pairs can be especially useful for configuring or processing data-driven logics widely used in AI systems. Such data can be readily generated from pre-existing radiological volumes and can provide more predictable quality and reliability. Moreover, these data can be generated with desired properties, such as artifacts, which can be helpful in providing more robust data-driven logics.

Thus, there can also be provided a use of the simulated radiological image and the image annotation data, as generated in any of the herein disclosed methods, for configuring (e.g., training and/or validating and/or evaluating) a data-driven or machine learning logic. Similarly, there can be provided a use of the radiological projection and the projection annotation data, as generated in any of the herein disclosed methods, for configuring (e.g., training and/or validating and/or evaluating) a data-driven or machine learning logic. Thus, for example, there can also be provided a method for improving a data-driven logic, comprising:
- training and/or validating and/or evaluating the data-driven logic via the simulated radiological image and the image annotation data and/or the radiological projection and the projection annotation data.

When viewed from another perspective, there can also be provided a logic or a memory storage or a computer storage medium storing the logic, at least partial computational functionality of which logic is caused by the logic being configured using the simulated radiological image and the image annotation data and/or the radiological projection and the projection annotation data as generated in any of the methods herein disclosed.

The term "configured using", in context of the use of data for data-driven techniques, it here means a logic having being trained and/or evaluated and/or validated using the above data.

"Data-driven logic" refers to a logic, which at least partially derives its functionality from training data.

When viewed from another perspective, there can also be provided a system comprising means for performing the steps of any of the herein disclosed methods.

For example, there can be provided a system comprising one or more computing units, wherein any one or more of the computing units are configured to:
- provide a three-dimensional radiological volume;
- provide volume annotation data; wherein the volume annotation data comprise a plurality of annotations, each annotation being indicative of an anatomical feature in the radiological volume,
- transform at least a part of the radiological volume to a radiological projection; wherein the radiological projection is a two-dimensional or planar representation of a part of the radiological volume,
- automatically provide, using the volume annotation data, projection annotation data for the radiological projection; wherein the projection annotation data comprise at least some of the annotations present in the volume annotation data.

When viewed from yet another perspective, there can also be provided a computer software product, or a non-transitory computer-readable storage medium storing the program, comprising instructions which when executed by a suitable one or more computing units cause any of the computing units to perform the steps of any of the herein disclosed methods.

For example, there can be provided a computer software product, or a non-transitory computer-readable storage medium storing the program, comprising instructions which when executed by a suitable one or more computing units cause any of the computing units to:
- provide a three-dimensional radiological volume;
- provide volume annotation data; wherein the volume annotation data comprise a plurality of annotations, each annotation being indicative of an anatomical feature in the radiological volume,
- transform the radiological volume to a plurality of radiological projections;
- automatically provide, using the volume annotation data, projection annotation data respectively for at least some of the radiological projections; wherein the projection annotation data of a given radiological projection comprise one or more projection annotations, each projection annotation being indicative of those one or more of the anatomical features present in that radiological projection,
- reconstruct, from the plurality of radiological projections, at least one simulated radiological image;
- generate, using the projection annotation data, image annotation data for at least one of the simulated radiological images; wherein the image annotation data comprise at least some of the annotations present in the volume annotation data.

"Computing unit", "computing device", "processing unit" or "processing device" may comprise, or it may be, a processing means or computer processor such as a microprocessor, microcontroller, or the likes, having one or more computer processing cores.

"Computer processor" refers to an arbitrary logic circuitry configured for performing basic operations of a computer or system, and/or, generally, to a device which is configured for performing calculations or logic operations. In particular, the processing means or computer processor may be configured for processing basic instructions that drive the computer or system. As an example, the processing means or computer processor may comprise at least one arithmetic logic unit ("ALU"), at least one floating point unit ("FPU"), such as a math coprocessor or a numeric coprocessor, a plurality of registers, specifically registers configured for supplying operands to the ALU and storing results of operations, and a memory, such as an L1 and L2 cache memory. In particular, the processing means or computer processor may be a multi core processor. Specifically, the processing means or computer processor may be or may comprise a central processing unit ("CPU"). the processing means or computer processor may be a complex instruction set computing ("CISC") microprocessor, reduced instruction set computing microprocessor ("RISC"), Very long instruction word ("VLIW") microprocessor, a processor implementing other instruction sets or processors implementing a combination of instruction sets. The processing means may also be one or more special purpose processing devices such as an application specific integrated circuit ("ASIC"), a field programmable gate array ("FPGA"), a complex programmable logic device ("CPLD"), a digital signal processor ("DSP"), a network processor, or the like. The methods, systems and devices disclosed herein may be implemented as software in a DSP, in a microcontroller, or in any other side processor such as hardware unit within an ASIC, CPLD, or FPGA. It is to be understood that the term processing means or processor may also refer to one or more processing devices, such as a distributed system of processing devices located across multiple computer systems (such as cloud computing), and is not limited to a single device unless otherwise specified.

"Connectivity interface" or "communication interface" refers to a software and/or hardware interface for establishing communication such as transfer or exchange of signals or data. The communication may either be wired, or it may be wireless. Connectivity interface is preferably based on or it supports one or more communication protocols. The communication protocol may be a wireless protocol, for example: short distance communication protocol such as Bluetooth^{®}, or Wi-Fi, or long communication protocols such as cellular or mobile network, for example, second generation cellular network ("2G"), 3G, 4G, long term evolution ("LTE"), or 5G. Alternatively, or in addition, the connectivity interface may even be based on proprietary short distance or long distance protocol. The connectivity interface may support any one or more standards and/or proprietary protocols.

"Network interface" refers to a device or a group of one or more hardware and/or software components that allow an operative connection with the network.

"Network" discussed herein may be any suitable kind of data transmission medium, wired, wireless, or their combination. A specific kind of network is not limiting to the scope or generality of the present teachings. The network can hence refer to any suitable arbitrary interconnection between at least one communication end point to another communication end point. Network may comprise one or more distribution points, routers or other types of communication hardware. The interconnection of the network may be formed by means of physically hard wiring, optical and/or wireless radio frequency ("RF") methods. The network specifically may be, or it may comprise, a physical network fully or partially made by hard wiring, such as a fiber optical network or a network fully or partially made by electrically conductive cables or a combination thereof. The network may at least partially comprise the Internet.

"Memory storage" may refer to a device for storage of information, in the form of data, in a suitable storage medium. Preferably, the memory storage is a digital storage suitable for storing the information in a digital form which is machine readable, for example digital data that are readable via a computer processor. The memory storage may thus be realized as a digital memory storage device that is readable by a computer processor. Further preferably, the memory storage on the digital memory storage device may also be manipulated by a computer processor. For example, any part of the data recorded on the digital memory storage device may be written and or erased and or overwritten, partially or wholly, with the new data by the computer processor.

That two or more components are "operatively" coupled or connected shall be clear to those skilled in the art. In a non-limiting manner, this means that there may be at least one communicative connection between the coupled or connected components e.g., they are the network interface or any suitable interface. The communicative connection may either be fixed, or it may be removable. Moreover, the communicative connection may either be unidirectional, or it may be bidirectional. Furthermore, the communicative connection may be wired and/or wireless. In some cases, the communicative connection may also be used for providing control signals.

### BRIEF DESCRIPTION OF THE SEVERAL VIEWS OF THE DRAWINGS

Certain aspects of the present teachings will now be discussed with reference to the accompanying drawings that explain said aspects by the way of examples. Since the generality of the present teachings is not dependent on it, the drawings may not be to scale. Method and system aspects may be discussed in conjunction for ease of understanding. Certain features shown in the drawings may be logical features that are shown together with physical features for the sake of understanding and without affecting the generality or scope of the present teachings.
FIG. 1 illustrates various transformations pursuant to the present teachings;
FIG. 2 illustrates a flowchart of an aspect of the present teachings;
FIG. 3 illustrates a flowchart of another aspect of the present teachings.

### DETAILED DESCRIPTION

In accordance with example aspects described herein, methods, systems and computer readable storage media can be provided, e.g., for improving a medical radiological process.

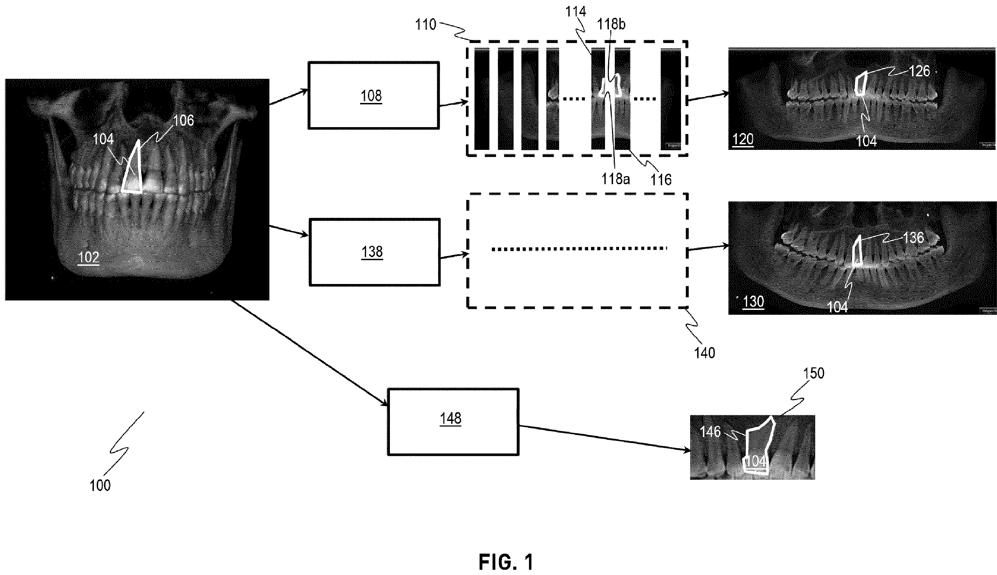

FIG. 1 shows a block diagram 100 illustrating some of the transformations of a radiological volume 102.

The radiological volume 102 here is shown as a 3D volume representation of craniofacial anatomy of a patient. The radiological volume 102 is showing a plurality of anatomical features such as teeth and bones of the patient's jaw. The radiological volume 102 is reconstructed from data acquired via a radiological scan of the patient. It is also provided volume annotation data 106, which are shown as an outline of one of the anatomical features shown here as a tooth 104. For ease of understanding, only one annotation is shown in this example, however in practical cases, several annotations, also of different types would be included in the volume annotation data 106. For example, volume annotation data 106 may include respective tooth number for some or all of the teeth. Similarly, other anatomical features such as bones, nerves, pathological conditions, and also information related to other anatomical parts, natural or artificial parts, may be provided in the volume annotation data 106. It shall be appreciated that the volume annotation data 106 may either be provided together with the radiological volume 102, or separately.

The radiological volume 102 is transformed, for example, via a transformation logic 108, to a plurality of radiological projections 110. The radiological projections may have an arbitrary aspect ratio, such as the thin and tall radiological projection 114 and radiological projection 116. Some of the radiological projections obtained by transforming the radiological volume 102 are shown in the box 110 representing the plurality of radiological projections. More specifically, it can be seen that the radiological projections 114 and 116 are provided with their respective projection annotation data 118a and 118b, which are automatically provided, e.g., via the transformation logic 108, wherever appropriate. It can be seen that each of the projection annotations 118a and 118b are indicative of the anatomical feature 104 which is present in these radiological projections 114 and 116. The transformation may be performed via a computer simulated physical scan of the radiological volume 102. Hence, the plurality of radiological projections 110 may be provided via an output of the physical scan. The physical scan may emulate properties of a real-life radiological system, such as exposure time, arm radius, scan path and so forth.

In this case, a simulated radiological image 120 is reconstructed, e.g., using a reconstruction logic (not explicitly shown in FIG. 1), from the plurality of radiological projections 110. The simulated radiological image 120 is provided image annotation data 126, which are generated using the projection annotation data 118a and 118b.

An advantage of doing so is that the annotations can be transformed more correctly as well as without user intervention, irrespective of the perspective in which the simulated radiological image 120 is reconstructed. This can be appreciated better by comparing with another simulated radiological image 130 which is obtained by reconstructing from another plurality of radiological projections 140. The another plurality of radiological projections 140 (slices in this case are not explicitly shown) is provided from the same radiological volume 102, e.g., by using a transformation logic 138 which may be the same as the transformation logic 108 with which the plurality of radiological projections 110 was provided, or it may be a different one. It can be seen that the perspective of the another simulated radiological image 130 is different from the perspective of the simulated radiological image 120. In this case, the pose of the volume 102 is different between the two simulated images 120 and 130, which corresponds to different poses of the simulated patient's head. Further, advantageously, it can be seen that the annotations are adapted to the perspective, for example, the another image annotation data 136 are different as compared to the image annotation data 126. In this case, the respective annotations automatically adapt to the shape and perspective of the tooth 104.

As a third variation, another radiological projection 150 is shown, which corresponds to a one-shot image of a part of the radiological volume 102. Unlike in the case of the simulated radiological image 120 and the another simulated radiological image 130, the another radiological projection 150 does not require a reconstruction operation. The another radiological projection 150 is shown here as a one-shot intraoral image. The another radiological projection 150 is provided projection annotation data 146 using the volume annotation data 106. Similar to the cases of the simulated radiological image 120 and the another simulated radiological image 130, the projection annotation data 146 are adapted to the perspective from which the another radiological projection 150 is captured from the radiological volume 102. Similar to the above cases, the transformation may be performed via a computer simulated physical one-shot scan of the radiological volume 102. Hence, the another radiological projection 150 may be provided via an output of the scan.

The images discussed above and their associated annotation data may either be provided at the same location or memory storage, and/or they may be provided at different ones.

Any one or more of the images discussed above, i.e., the simulated radiological image 120, the another simulated radiological image 130 and the another radiological projection 150 may be a part of one or more datasets which are used for processing, i.e., configuring or improving a data-driven logic, for example, training, validating and/or evaluating a data-driven logic.

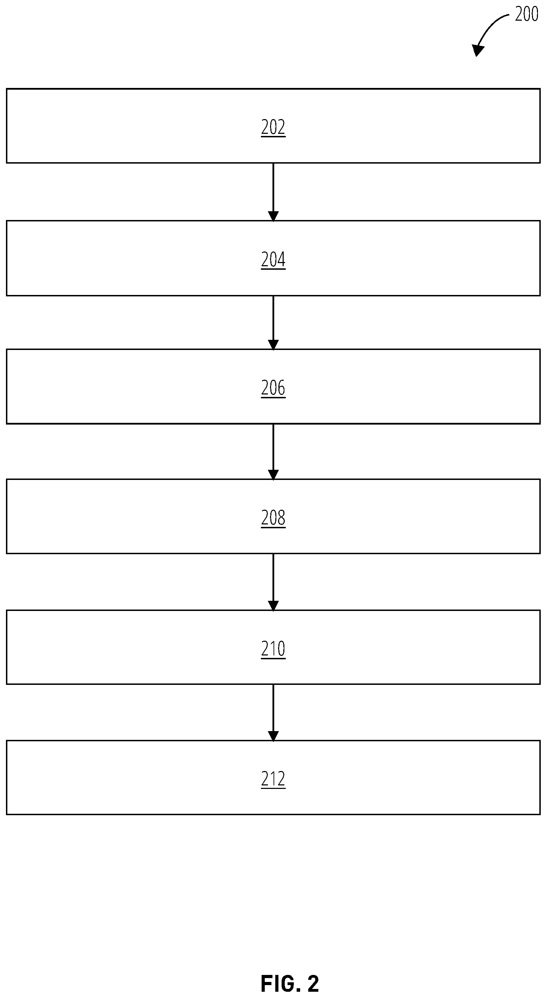

FIG. 2 shows a flowchart 200 exemplifying an aspect of the present teachings. The flowchart 200 can be implemented as a routine executable by one or more computing units, for example, operatively connected to a radiology apparatus, for example, a CBCT system for dental applications.

In block 202, it is provided a three-dimensional radiological volume 102. The radiological volume 102 may be in the form of CT volume, CBCT, DVT volume, or their likes.

In block 204, it is provided volume annotation data 106. The volume annotation data 106 comprise a plurality of annotations. Each annotation is indicative of a respective anatomical feature, e.g., the annotation 106 is indicative of a specific tooth 104 in the radiological volume 102.

In block 206, the radiological volume 102 is transformed to one or more radiological projections 114 or to an another radiological projection 150.

In block 208, it is automatically provided projection annotation data 118a and 118b respectively for at least some of the radiological projections, e.g., 114, 116, and 150. For example, the projection annotation data 118a of the radiological projection 114 comprises at least one annotation indicative of the at least one anatomical feature 104 present in the radiological projection 114. Similarly, the projection annotation data 146 of the another radiological projection 150 comprises at least one annotation indicative of the at least one anatomical feature 104 present in the another radiological projection 150.

In block 210, it is reconstructed a simulated radiological image 120 from the plurality of radiological projections 110.

In block 212, it is generated image annotation data 126 from the projection annotation data 118a and 118b. The image annotation data 126 comprise at least some of the annotations present in the volume annotation data 106.

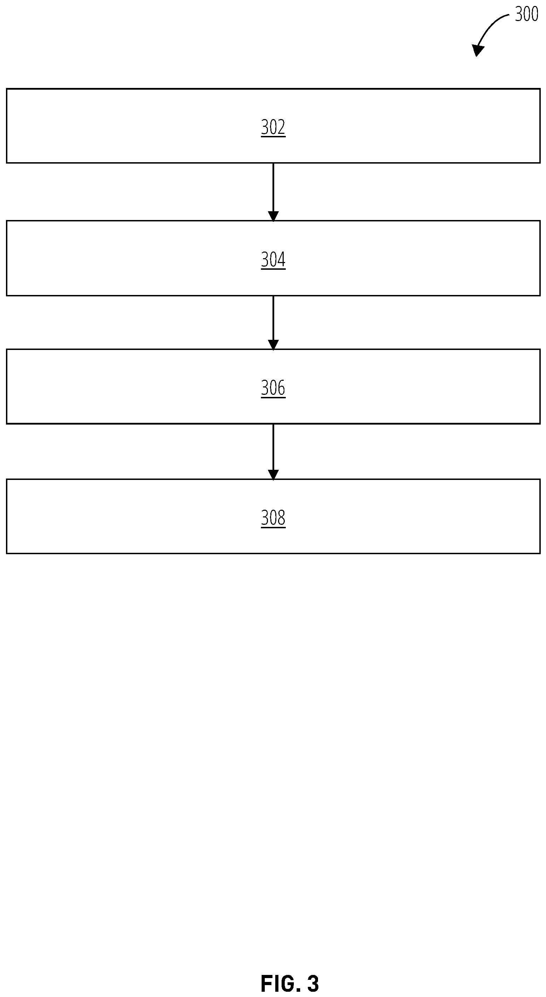

FIG. 3 shows another flowchart 300 exemplifying another aspect of the present teachings, for specifically showing the one-shot image process. The flowchart 300 can be implemented as a routine executable by one or more computing units, for example, operatively connected to a radiology apparatus, for example, a CBCT system for dental applications.

In block 302, it is provided a 3D radiological volume 102. The radiological volume 102 may be in the form of CT volume, CBCT, DVT volume, or their likes.

In block 304, it is provided volume annotation data 106. The volume annotation data 106 comprise a plurality of annotations. Each annotation is indicative of a respective anatomical feature, e.g., the annotation 106 is indicative of a specific tooth 104 in the radiological volume 102.

In block 306, the radiological volume 102 is transformed to a radiological projection 150. The radiological projection 150 is a two-dimensional or planar representation of a part of the radiological volume 102.

In block 308, it is automatically provided projection annotation data 146 for the radiological projection 150. The projection annotation data 146 of the radiological projection 150 comprises at least one annotation indicative of the at least one anatomical feature 104 present in the volume annotation data 106.

The method steps may be performed in the order as shown listed in the examples or aspects. It should be noted, however, that under specific circumstances a different order may also be possible. Further, it is also possible to perform one or more of the method steps once or repeatedly. These steps may be repeated at regular or irregular time periods. Further, it is possible to perform two or more of the method steps simultaneously or in a timely overlapping fashion, specifically when some or more of the method steps are performed repeatedly. The method may comprise further steps which are not listed.

The word "comprising" does not exclude other elements or steps, and the indefinite article "a" or "an" does not exclude a plurality. A single processing means, processor or controller or other similar unit may fulfill the functions of several items recited in the claims. The mere fact that certain measures are recited in mutually different dependent claims does not indicate that a combination of these measures cannot be used to advantage. Any different signs in the claim should not be construed as limiting the scope.

Further, it should be noted that in the present disclosure, the terms "at least one", "one or more" or similar expressions indicating that a feature or element may be present once or more than once typically may have been used only once when introducing the respective feature or element. Thus, in some cases unless specifically stated otherwise, when referring to the respective feature or element, the expressions "at least one" or "one or more" may not have been repeated, notwithstanding the fact that the respective feature or element may be present once or more than once.

Further, the terms "preferably", "more preferably", "particularly", "more particularly", "specifically", "more specifically" or similar terms are used in conjunction with optional features, without restricting alternative possibilities. Thus, any features introduced by these terms are optional features and are not intended to restrict the scope of the claims in any way. The present teachings may, as those skilled in the art will recognize, be performed by using alternative features. Similarly, the features introduced by "according to an aspect" or similar expressions are intended to be optional features, without any restriction regarding alternatives to the present teachings, without any restrictions regarding the scope of the present teachings and without any restriction regarding the possibility of combining the features introduced in such way with other optional or non-optional features of the present teachings.

Unless otherwise defined, all technical and scientific terms used herein have the same meaning as commonly understood by one of ordinary skill in the art to which the present teachings belong.

Various examples have been disclosed above for a method, a system, a device, a use, software program, and a computing unit comprising the computer program code for carrying out the methods herein disclosed. For example, it has been disclosed a method for improving a medical radiological process, comprising: providing a three-dimensional radiological volume; providing volume annotation data; wherein the volume annotation data comprise a plurality of annotations, transforming the radiological volume to a plurality of radiological projections; automatically providing, using the volume annotation data, projection annotation data for at least some of the radiological projections; reconstructing, from the plurality of radiological projections, a simulated radiological image; generating, using the projection annotation data, image annotation data. The present teachings also relate to systems, software products, storage media and uses. Those skilled in the art will understand however that changes and modifications may be made to those examples without departing from the accompanying claims. It will further be appreciated that aspects from the method and product embodiments discussed herein may be freely combined.

Any headings utilized within the description are for convenience only and have no legal or limiting effect.

## Claims

1. A computer-implemented method of dental radiography for improving a medical radiological process, said method comprising the steps of:
- providing (202) a three-dimensional radiological volume;
- providing (204) volume annotation data; wherein the volume annotation data comprise a plurality of annotations, each annotation being indicative of an anatomical feature in the radiological volume,
- transforming (206) at least a part of the radiological volume to a plurality of radiological projections; wherein each radiological projection is a two-dimensional or planar representation of a part of the radiological volume,
- automatically providing (208), using the volume annotation data, projection annotation data for the radiological projection; wherein the projection annotation data comprise at least some of the annotations present in the volume annotation data; and
- reconstructing (210), from said plurality of radiological projections, one or more simulated radiological images, wherein said simulated radiological image is one of the following: a panoramic image reconstruction, a DVT reconstruction, a CEPH reconstruction, bitewing image reconstruction; and
- generating (212), using the projection annotation data, image annotation data for the simulated radiological image; wherein the image annotation data of each of the simulated radiological images comprise at least some of the annotations present in the volume annotation data.

2. The method of claim 1, wherein the transformation of the radiological volume involves:
- performing a simulated physical scan of the three-dimensional radiological volume;
- generating, from the simulated scan, the radiological projection or the plurality of radiological projections.

3. The method of any one of claims 1 to 2, wherein the volume annotation data comprise one or more voxel segmentation masks and/or one or more outlines and/or one or more point landmarks.

4. The method of any one of claims 1 to 3, wherein the projection annotation data comprise one or more voxel segmentation masks and/or pixel segmentation masks and/or one or more point landmarks.

5. The method of any one of claims 1 to 4, further comprising:
- performing a data processing operation on at least some of the radiological projections and/or the simulated radiological image; wherein, the data processing operation comprises any one or more of: contrast enhancement, providing artifact data, averaging, and filtering.

6. The method of any one of claims 1 to 5, wherein transforming the radiological volume to at least one of the radiological projections further comprises including one or more non-idealities, wherein the non-ideality causes one or more simulated artifacts in at least some of the radiological projections and/or simulated radiological images.

7. A system comprising a processor adapted to perform the steps of any of the above method claims.

8. A computer software product, or a non-transitory computer-readable storage medium storing a program, comprising instructions which when executed by a suitable one or more computing units cause any of the computing units to perform the steps of any of the claims 1-6.

## Patentansprüche

1. Computerimplementiertes Verfahren zur zahnärztlichen Radiographie zum Verbessern eines medizinischen radiologischen Prozesses, wobei das Verfahren die folgenden Schritte umfasst:
- Bereitstellen (202) eines dreidimensionalen radiologischen Volumens;
- Bereitstellen (204) von Volumenbeschriftungsdaten; wobei die Volumenbeschriftungsdaten eine Vielzahl von Beschriftungen umfassen, wobei jede Beschriftung für ein anatomisches Merkmal in dem radiologischen Volumen bezeichnend ist,
- Transformieren (206) zumindest eines Teils des radiologischen Volumens in eine Vielzahl von radiologischen Projektionen; wobei jede radiologische Projektion eine zweidimensionale oder ebene Darstellung eines Teils des radiologischen Volumens ist,
- automatisches Bereitstellen (208), unter Verwendung der Volumenbeschriftungsdaten, von Projektionsbeschriftungsdaten für die radiologische Projektion; wobei die Projektionsbeschriftungsdaten mindestens einige der Beschriftungen umfassen, die in den Volumenbeschriftungsdaten vorhanden sind; und
- Rekonstruieren (210), aus der Vielzahl von radiologischen Projektionen, von einem oder mehreren simulierten radiologischen Bildern, wobei das simulierte radiologische Bild eines der Folgenden ist: eine Panoramabildrekonstruktion, eine DVT-Rekonstruktion, eine CEPH-Rekonstruktion, Bissflügelbildrekonstruktion; und
- Erzeugen (212), unter Verwendung der Projektionsbeschriftungsdaten, von Bildbeschriftungsdaten für das simulierte radiologische Bild; wobei die Bildbeschriftungsdaten von jedem der simulierten radiologischen Bilder zumindest einige der Beschriftungen umfassen, die in den Volumenbeschriftungsdaten vorhanden sind.

2. Verfahren nach Anspruch 1, wobei die Transformation des radiologischen Volumens Folgendes involviert:
- Durchführen eines simulierten physikalischen Scans des dreidimensionalen radiologischen Volumens;
- Erzeugen der radiologischen Projektion oder der Vielzahl von radiologischen Projektionen aus dem simulierten Scan.

3. Verfahren nach einem der Ansprüche 1 bis 2, wobei die Volumenbeschriftungsdaten eine oder mehrere Voxelsegmentierungsmasken und/oder eine oder mehrere Umrisse und/oder eine oder mehrere Punktlandmarken umfassen.

4. Verfahren nach einem der Ansprüche 1 bis 3, wobei die Projektionsbeschriftungsdaten eine oder mehrere Voxelsegmentierungsmasken und/oder Pixelsegmentierungsmasken und/oder eine oder mehrere Punktlandmarken umfassen.

5. Verfahren nach einem der Ansprüche 1 bis 4, ferner umfassend:
- Durchführen eines Datenverarbeitungsvorgangs an zumindest einigen der radiologischen Projektionen und/oder des simulierten radiologischen Bildes; wobei der Datenverarbeitungsvorgang eines oder mehrere vom Folgenden umfasst: Kontrastverbesserung, Bereitstellen von Artefaktdaten, Mitteln und Filtern.

6. Verfahren nach einem der Ansprüche 1 bis 5, wobei das Transformieren des radiologischen Volumens in mindestens eine der radiologischen Projektionen ferner das Enthalten einer oder mehrerer Nicht-Idealitäten umfasst, wobei die Nicht-Idealität ein oder mehrere simulierte Artefakte in zumindest einigen der radiologischen Projektionen und/oder simulierten radiologischen Bilder verursacht.

7. System, umfassend einen Prozessor, der dazu angepasst ist, die Schritte nach einem der obigen Verfahrensansprüche durchzuführen.

8. Computersoftwareprodukt oder nicht-flüchtiges computerlesbares Speichermedium, das ein Programm speichert, umfassend Anweisungen, die, wenn sie durch eine geeignete eine oder mehrere Recheneinheiten ausgeführt werden, verursachen, dass beliebige der Recheneinheiten die Schritte nach einem der Ansprüche 1 bis 6 durchführen.

## Revendications

1. Procédé de radiographie dentaire mis en œuvre par ordinateur pour améliorer un processus radiologique médical, ledit procédé comprenant les étapes de :
- fourniture (202) d'un volume radiologique tridimensionnel ;
- fourniture (204) de données d'annotation de volume ; dans lequel les données d'annotation de volume comprennent une pluralité d'annotations, chaque annotation indiquant une caractéristique anatomique dans le volume radiologique,
- transformation (206) d'au moins une partie du volume radiologique en une pluralité de projections radiologiques ; dans lequel chaque projection radiologique est une représentation bidimensionnelle ou plane d'une partie du volume radiologique,
- fourniture automatique (208), à l'aide des données d'annotation de volume, de données d'annotation de projection pour la projection radiologique ; dans lequel les données d'annotation de projection comprennent au moins certaines des annotations présentes dans les données d'annotation de volume ; et
- reconstruction (210), à partir de ladite pluralité de projections radiologiques, d'une ou de plusieurs images radiologiques simulées, dans lequel ladite image radiologique simulée est l'une des suivantes : une reconstruction d'image panoramique, une reconstruction DVT, une reconstruction CEPH, une reconstruction d'image bitewing ; et
- génération (212), à l'aide des données d'annotation de projection, de données d'annotation d'image pour l'image radiologique simulée ; dans lequel les données d'annotation d'image de chacune des images radiologiques simulées comprennent au moins certaines des annotations présentes dans les données d'annotation de volume.

2. Procédé de la revendication 1, dans lequel la transformation du volume radiologique implique :
- la réalisation d'un balayage physique simulé du volume radiologique tridimensionnel ;
- la génération, à partir du balayage simulé, de la projection radiologique ou de la pluralité de projections radiologiques.

3. Procédé de l'une quelconque des revendications 1 ou 2, dans lequel les données d'annotation de volume comprennent un ou plusieurs masques de segmentation de voxels et/ou un ou plusieurs contours et/ou un ou plusieurs points de repère.

4. Procédé de l'une quelconque des revendications 1 à 3, dans lequel les données d'annotation de projection comprennent un ou plusieurs masques de segmentation de voxels et/ou masques de segmentation de pixels et/ou un ou plusieurs points de repère.

5. Procédé de l'une quelconque des revendications 1 à 4, comprenant en outre :
- la réalisation d'une opération de traitement de données sur au moins certaines des projections radiologiques et/ou l'image radiologique simulée ; dans lequel l'opération de traitement de données comprend une ou plusieurs opérations quelconques parmi : l'amélioration du contraste, la fourniture de données d'artefact, le moyennage et le filtrage.

6. Procédé de l'une quelconque des revendications 1 à 5, dans lequel la transformation du volume radiologique en au moins l'une des projections radiologiques comprend en outre l'inclusion d'une ou de plusieurs non-idéalités, dans lequel la non-idéalité entraîne un ou plusieurs artefacts simulés dans au moins certaines des projections radiologiques et/ou des images radiologiques simulées.

7. Système comprenant un processeur adapté pour réaliser les étapes de l'une quelconque des revendications de procédé ci-dessus.

8. Produit logiciel informatique, ou support de stockage lisible par ordinateur non transitoire stockant un programme, comprenant des instructions qui, lorsqu'elles sont exécutées par une ou plusieurs unités de calcul appropriées, amènent l'une quelconque des unités de calcul à réaliser les étapes de l'une quelconque des revendications 1 à 6.
